# EUROPÄISCHE PATENTSCHRIFT

(11) **EP 3 027 117 B1**
(45) Veröffentlichungstag und Bekanntmachung des Hinweises auf die Patenterteilung: **31.10.2018**
(21) Anmeldenummer: 14744554.8
(22) Anmeldetag: 28.07.2014
(51) Int. Cl.: A61B 17/04, A61B 17/06, A61F 2/00

(54) **ANORDNUNG ZUM EINSETZEN EINES CHIRURGISCHEN IMPLANTATS MIT MINDESTENS EINEM IMPLANTATANSATZABSCHNITT**
ARRANGEMENT FOR INSERTING A SURGICAL IMPLANT WITH AT LEAST ONE IMPLANT ATTACHMENT PORTION
DISPOSITIF D'INSERTION D'UN IMPLANT CHIRURGICAL COMPORTANT AU MOINS UN SEGMENT D'INSERTION D'IMPLANT

(30) Priorität: 29.07.2013 DE 202013103406 U
(43) Veröffentlichungstag der Anmeldung: 08.06.2016
(73) Patentinhaber: PFM Medical AG, 50996 Köln (DE)
(72) Erfinder: ZIMMERMANN, Hanngörg, 91327 Gössweinstein (DE)
(74) Vertreter: Hohendorf, Carsten
(86) Internationale Anmeldenummer: PCT/EP2014/066201
(87) Internationale Veröffentlichungsnummer: WO 2015/014806

(56) Entgegenhaltungen:
- US-A- 5 152 749
- US-A1- 2006 058 575
- US-A1- 2008 091 058
- US-A1- 2008 234 543

## Beschreibung

Die Erfindung betrifft eine Anordnung zum Einsetzen eines chirurgischen Implantats mit mindestens einem Implantatansatzabschnitt, insbesondere zum Einsetzen von Implantaten im Bereich der chirurgischen Beckenbodenrekonstruktion.

Die Anatomie des Beckenbodens ist sehr komplex und insbesondere bei Fasziendefekten variabel. Chirurgische Eingriffe erfordern ein extrem hohes Niveau an chirurgischen Fähigkeiten, da der Zugriff von einem trans-vaginalen Ansatz tief in der Beckenhöhle erfolgt. Ein Vernähen tief in der Beckenhöhle ist daher schwierig und sehr zeitaufwendig. Weiterhin erfordern Beckenbodenreparaturoperationen ein Schneiden und/oder Zerlegen in unmittelbarer Nähe zu kritischen Strukturen wie beispielsweise der Blase, dem Rektum, dem Peritoneum und einer Vielzahl von wesentlichen Gefäß- und Nervenstrukturen.

Aus dem Stand der Technik ist es bekannt synthetische oder biologische Netzimplantate für eine chirurgische Reparatur von beschädigtem Bindegewebe, insbesondere Faziengewebe wie beispielsweise einer Hernie zu verwenden. Die in diesem Zusammenhang entwickelten Materialien und Techniken werden weiterhin zur Reparatur des weiblichen Beckenbodens bei Defekten wie beispielsweise Zystozelen, Rektozelen, Enterozelen, zur Behandlung einer Harninkontinenz und Vorfällen des Scheidengewölbes eingesetzt.

Die aus dem Stand der Technik bekannten Implantate umfassen einen Mittelteil und eine Mehrzahl von bandartigen Erweiterungen auf, wobei die bandartigen Erweiterungen als Implantatansatzabschnitte dienen. Üblicherweise weisen derartige Implantate 2, 4 oder 6 Implantatansatzabschnitte auf. Zur Behandlung einer Harninkontinenz werden auch bandförmige Implantate eingesetzt, wobei die beiden Enden des Bandes als Implantatansatzabschnitte dienen.

Die US 2003/0220538 offenbart ein Verfahren und ein entsprechendes Implantat für eine Implantation von Implantaten im Bereich der Beckenbodenrekonstruktion. Das beschriebene Verfahren beseitigt die Notwendigkeit zum Befestigen des Implantats durch Nähen mittels bandartiger Netzmaterialerweiterungen (Implantatansatzabschnitte), die durch das Sakrospinalband und/oder die Obteratormembran hindurchtreten.

Das Dokument US 2004/0039453 betrifft die Behandlung einer Zystozele mittels eines Implantats umfassend Bänder.

Die EP 1 333 774 B1 offenbart zur Behandlung einer Harninkontinenz eine Anordnung zum Einführen eines Bandes in den Bauchraum einer Frau durch die Vagina, dass als pubovaginale Schlinge bei der Behandlung von durch Belastung ausgelöster Harninkontinenz dient, wobei die Vorrichtung aufweist:
ein Paar gebogener, im Wesentlichen starrer Zuführnadeln, die für das beidseitige Einführen in den Bauchraum einer Frau zur Anordnung auf jeder Seite des Blasenhalses benachbart der Harnröhre ausgelegt sind, wobei die Zuführnadeln gemeinsam geeignet sind, einen Zuführweg für das Band zu definieren;
eine Befestigungseinrichtung für das lösbare Befestigen des proximalen Endes jeder aus dem Paar der Zuführnadeln an einem Ende des Bandes, dass bei der Verwendung durch die Vagina eingeführt worden ist; und
ein Paar gebogener, schlauchförmiger im Wesentlichen flexibler Zuführhülsen, wobei jede aus dem Paar der Zuführhülsen ein distales Hülsenende und ein proximales Hülsenende hat, die einen Zuführhülsendurchlass definieren, der in deren Inneren angeordnet ist und dazu ausgelegt ist, in den Zuführweg über ihr proximales Hülsenende eingefügt zu werden und entfernbar eine aus dem Paar der Zuführnadeln aufzunehmen, wobei jede aus dem Paar der Zuführnadeln dazu ausgelegt ist, durch den Zuführhülsendurchlass aus dem Bauchraum zurückgezogen zu werden, so dass ein Ende des Bandes von der Vagina durch den Zuführhülsendurchlass von dem proximalen Hülsenende zu dem distalen Hülsenende geleitet wird, so dass das Band entlang dem Zuführweg angeordnet wird.

Aus der EP 1 804 715 B1 ist eine Anordnung zum Einsetzen eines chirurgischen Implantats mit mindestens einem Implantatansatzabschnitt bekannt, wobei die Anordnung umfasst:
ein Führungselement mit einem distalen Gewebedurchbohrende;
eine Kanüle mit einem proximalen Ende, einem distalen Ende und einem Kanal, der dort hindurch verläuft und in dem das Führungselement gleitfähig aufnehmbar ist derart, dass die Gewebedurchbohrspitze des Führungselements vom distalen Ende der Kanüle nach außen verläuft;
eine Zurückholeinrichtung mit einem Fangelement an einem distalen Ende derselben zum Koppeln mit dem Implantatansatzabschnitt des chirurgischen Implantats;
wobei die Kanüle mit dem darin gleitfähig aufgenommenen Führungselement so dimensioniert ist, dass sie durch die Beckenhöhle eines Patienten von einem äußeren Einschnitt und durch einen vaginalen Einschnitt heraus verläuft, und der Kanülenkanal für einen Durchgang der Zurückholeinrichtung und des angekoppelten Implantatansatzabschnitts durch selbigen dimensioniert ist. Die Erfindung gemäß der EP 1 804 715 B1 zeichnet sich dadurch aus, dass die Kanüle ferner eine elastische Endregion enthält, wobei die elastische Endregion eine erste Gestalt annimmt, wenn darin das Führungselement aufgenommen ist, und eine zweite Gestalt annimmt, wenn das Führungselement aus der Kanüle entfernt ist, wobei die zweite Gestalt eine größere Krümmung als die erste aufweist.
Aus der US 2008/234543 A1 ist eine Anordnung entsprechend des Oberbegriffs des unabhängigen Anspruchs bekannt.

Bei dem Einsetzen von Netzimplantaten mit mindestens einem bandartigen Implantatansatzabschnitt zur Beckenbodenrekonstruktion oder Behandlung einer Harninkontinenz wird zunächst ein äußerer Einschnitt im Bereich des Implantationsorts ausgeführt. Zum Fixieren des Netzimplantats wird in einem ersten Schritt das Führungselement durch den Kanal der Kanüle geführt, bis das Gewebedurchbohrende aus dem distalen Ende der Kanüle austritt. Nachfolgend wird das Führungselement zusammen mit der Kanüle durch den äußeren Einschnitt eingeführt und durch das umliegende Gewebe geführt, bis das Gewebedurchbohrende des Führungselements und das distale Ende der Kanüle in die Vagina der Patientin austreten. Als nächstes wird das Führungselement aus der Kanüle entfernt, wobei die Lage der Kanüle nicht verändert wird. Durch den Kanal der Kanüle wird nun die Zurückholeinrichtung in die Patientin eingeführt. Dieser Vorgang wird für jeden Implantatansatzabschnitt des einzusetzenden Implantats wiederholt. Beim Einsetzten eines Implantats mit sechs Implantatansatzabschnitten wird der erste Schnitt beispielsweise an der genitofermoralen Falte am lateralen Rand des absteigenden Schambeinastes auf der Höhe der Klitoris ausgeführt. Der zweite Einschnitt wird 1 bis 2 cm lateral und 2 bis 3 cm caudal vom ersten ausgeführt. Der dritte Einschnitt wird 3 cm lateral und dorsal vom Anus ausgeführt. Dieselben Einschnitte werden auf der gegenüberliegenden Seite ausgeführt. Die Implantatansatzabschnitte des einzusetzenden Implantats werden mit den Fangelementen der Zurückholeinrichtungen eingefangen. Sobald die Implantatansatzabschnitte mit dem Fangelementen der Zurückholeinrichtungen gekoppelt sind, werden die Zurückholeinrichtungen aus den Kanälen der entsprechenden Kanülen entfernt, so dass sich die gekoppelten Implantatansatzabschnitte durch die Kanäle der entsprechenden Kanülen erstrecken. Anschließend werden die Kanüle entfernt, wobei die Implantatansatzabschnitte aus den Kanälen der entsprechenden Kanülen entfernt werden, so dass sich die Implantatansatzabschnitte weiterhin zwischen dem äußeren ersten Einschnitt und dem inneren der Vagina der Patientin erstrecken. Das Implantat wird mittels der Implantatansatzabschnitte so innerhalb der Vagina platziert, dass es das geschwächte oder beschädigte Bindegewebe unterstützt. Nach dem Einsetzen des Implantats verwachsen die Implantatansatzabschnitte mit dem umliegenden Gewebe, wodurch das Implantat an der gewünschten Stelle fixiert wird. Die Verwendung von Kanülen hat dabei den Vorteil, dass das einzusetzende Implantat einfach justiert und platziert werden kann, da zwischen den Kanälen der Kanülen und den Implantatansatzabschnitten kaum Reibungskräfte auftreten.
Der vorliegenden Erfindung liegt die **Aufgabe** zugrunde, die aus dem Stand der Technik bekannten Instrumente zum Einsetzen eines chirurgischen Implantats mit mindestens einem Implantatansatzabschnitt zu verbessern, insbesondere hinsichtlich des Einfangens der Implantatansatzabschnitte mit dem Fangelement der Zurückholeinrichtung.
Die Aufgabe wird erfindungsgemäß **gelöst** durch eine Anordnung zum Einsetzen eines chirurgischen Implantats mit mindestens einem Implantatansatzabschnitt entsprechend den Merkmalen des unabhängigen Anspruchs.

Die aus dem Stand der Technik der Technik bekannten Kanülen sind in dem zweiten Zustand gekrümmt ausgebildet, insbesondere weist die Kanüle gemäß der EP 1 804 715 B1 in dem zweiten Zustand eine größere Krümmung auf als in dem ersten Zustand. Dadurch soll erreicht werden, dass das distale Ende der Kanüle in Richtung des Operateurs weist, wenn dieses in den Hohlraum des menschlichen Körpers, insbesondere der Vagina, austritt. Dies bedingt jedoch eine lagegenaue Anordnung der Kanüle relativ zu dem Führungselement und dem radialen Einführwinkel in den äußeren Einschnitt. Tritt das distale Ende der Kanüle nicht in einem bestimmten Winkel in den Hohlraum des menschlichen Körpers, insbesondere der Vagina, aus, so wendet sich das distale Ende nach dem Entfernen des Führungselement aus dem Kanal der Kanüle von dem Operateur ab, wodurch ein nachfolgendes Einfangen eines Implantatansatzabschnitts mit dem Fangelement der Zurückholeinrichtung deutlich erschwert wird. Erfindungsgemäß weist die Kanüle in dem zweiten Zustand eine im Wesentlichen gerade Form auf. Somit tritt das distale Ende der Kanüle unabhängig von der Ausrichtung der Kanüle zu dem Führungselement und weiterhin unabhängig von dem radialen Einführwinkel in den äußeren Einschnitt immer in derselben Richtung in den Hohlraum des menschlichen Körpers, insbesondere der Vagina, aus, so dass insbesondere mit zunehmender Erfahrung des Operateurs das Einfangen des Implantatansatzabschnitts mittels des Fangelements der Zurückholeinrichtung erleichtert wird.
Dass die Kanüle in dem zweiten Zustand im Wesentlichen gerade ausgebildet ist, bedeutet im Sinne der Erfindung, dass zumindest das distale Ende der Kanüle, welches in den Hohlraum des menschlichen Körpers, insbesondere der Vagina, austritt, gerade ausgebildet ist.

Nach einer Variante der Erfindung ist der Durchmesser des Kanals der Kanüle am proximalen Ende der Kanüle erweitert. Dadurch lässt sich das Führungselement oder die Zurückholeinrichtung einfacher in den Kanal der Kanüle einführen. Gemäß einer zweckmäßigen Variante der Erfindung weist die Kanüle am proximalen Ende wenigstens einen Handgriff auf. Dadurch lässt sich die Kanüle während des Einsatzes einfacher handhaben. Weiterhin verhindert der Handgriff, dass die komplette Kanüle durch den äußeren Einschnitt hindurchtritt. Vorzugsweise besteht der wenigstens eine Handgriff aus einem starren Material, beispielsweise einem starren Kunststoff oder einem Metall.
Eine weitere Variante der Erfindung zeichnet sich dadurch aus, dass sich der Außendurchmesser der Kanüle am distalen Ende konisch verringert. Dadurch wird ein möglichst fließender Übergang zwischen Führungselement und Kanüle erreicht, wodurch sich die Kanüle zusammen mit dem Führungselement einfacher durch den äußeren Einschnitt und das umliegende Gewebe in den Hohlraum des menschlichen Körpers, insbesondere der Vagina, einführen lässt.
In einer weiteren erfindungsgemäßen Variante verringert sich der Durchmesser des Kanals am distalen Ende, zweckmäßigerweise auf einen Durchmesser der etwas geringer ist als der Außendurchmesser des Führungselements am distalen Ende, jedoch größer als der Durchmesser des Gewebedurchbohrendes des Führungselements. Dadurch wird die Kanüle mittels einer reibschlüssigen Verbindung beim Einführen in den äußeren Einschnitt relativ zu dem Führungselement fixiert.
Zweckmäßigerweise besteht die Kanüle aus wenigstens einem Kunststoff. Zur Verbesserung der Steifigkeit bzw. als Knickschutz sind innerhalb des Kunststoffs ein oder mehrere Versteifungselemente, beispielsweise Metalldrähte, eingearbeitet werden.
Gemäß einer erfindungsgemäßen Variante ist das Fangelement der Zurückholeinrichtung als Schlinge oder Haken ausgebildet. Insbesondere mittels einer Schlinge lässt der Implantatansatzabschnitt auf einfache Art und Weise einfangen. Weiterhin nutzen Operateure Fangschlingen bei der Bergung von Fremdkörpern aus Hohlräumen oder Gefäßen innerhalb des menschlichen oder tierischen Körpers, so dass die Operateure den Umgang mit Fangschlingen gewohnt sind.

Vorzugsweise besteht das Fangelement der Zurückholeinrichtung aus einem Metall, Kunststoff oder Formgedächtnismaterial, insbesondere aus Edelstahl oder Nitinol.

Nach einer weiteren Variante der Erfindung ist das Fangelement derart ausgebildet, dass es sich nach dem Austreten aus dem distalen Ende des Kanals der Kanüle relativ zu der durch den Kanal der Kanüle verlaufenden Längsachse abwinkelt, vorzugsweise um etwa 90°. Somit liegt die durch das Fangelement verlaufende Ebene in etwa parallel zu der Oberfläche des Hohlraums des menschlichen Körpers, insbesondere der Vagina, wo die Kanüle in den Hohlraum des menschlichen Körpers, insbesondere der Vagina, austritt. Dadurch wird das Einfangen des wenigstens einen Implantatansatzabschnitts deutlich vereinfacht.

In einer Variante der Erfindung wird das Fangelement durch wenigstens einen durch die Zurückholeinrichtung verlaufenden Draht gebildet, welcher beispielsweise am distalen Ende der Zurückholeinrichtung eine Schlaufe ausbildet. Die Fangvorrichtung ist dabei innerhalb der Zurückholeinrichtung bewegbar. Damit sich die Fangvorrichtung nicht ungewollt relativ zu der Zurückholeinrichtung bewegt, weist die Zurückholeinrichtung nach einer erfindungsgemäßen Variante am proximalen Ende einen Knoten auf. Erst wenn der Knoten gelöst wurde kann die Fangvorrichtung relativ zu der Zurückholeinrichtung bewegt werden.

Gemäß einer weiteren Variante ist das Fangelement als Schlinge ausgebildet, wobei die Schlinge mittels einer distalen Manschette ausgebildet wird, in welcher ein Ende des die Schlinge ausbildenden Drahts fixiert ist, während ein anderer Bereich des Drahts gleitfähig in der distalen Manschette gelagert ist. Dadurch lässt sich die Größe der durch den Draht ausgebildeten Schlinge variieren. Insbesondere lässt sich der Durchmesser Schlinge nach dem Einfangen des Implantatansatzabschnitts derart verringern, dass der Implantatansatzabschnitt in der Schlinge fixiert wird. Zweckmäßigerweise ist an dem das Fangelement ausbildenden Draht ein Anschlag angeordnet, welche zusammen mit der Manschette die Schlingengröße begrenzt, wenn der Anschlag an die distale Manschette anschlägt. Die Schlinge wird somit in der maximalen Größe aus der Zurückholeinrichtung austreten und nach erfolgreichem Einfangen des Implantatansatzabschnitts zunächst in der Größe verringert, bevor die Schlinge in die Zurückholeinrichtung zurückgezogen wird.

Nach einer weiteren Variante der Erfindung ist das proximale Ende des Fangelements an einer proximalen Manschette fixiert, während ein anderer Bereich des Fangelements gleitfähig in der proximalen Manschette gelagert ist. Dadurch lässt sich die Gesamtlänge des Fangelements anpassen. Beim Einführen des Fangelements kann die am proximalen Ende ausgebildete Schlaufe verringert und beim Herausziehen vergrößert werden. Dadurch verringert sich der proximale Platzbedarf der Fangschlingen. Insbesondere wird die Gefahr eines Verhakens mit anderen während der Operation verwendeten Gegenständen und/oder bei der Operation anwesenden Personen verringert.

Zweckmäßigerweise ist das Implantat ein Netz zur Beckenbodenreparatur, ein Netz zur Zystozelenreparatur, ein Netz zur Rektozelenreparatur oder ein Netz, insbesondere ein bandartiges Netz, zur Behandlung einer Harninkontinenz.

Nach einer erfindungsgemäßen Variante ist der äußere Einschnitt ein Einschnitt im Unterbauch, Anusbereich oder im Oberschenkel in der Nähe des foramen obturatum der Patientin. Beim Einsetzten eines Implantats mit sechs Implantatansatzabschnitten wird der erste Schnitt beispielsweise an der genitofermoralen Falte am lateralen Rand des absteigenden Schambeinastes auf der Höhe der Klitoris ausgeführt. Der zweite Einschnitt wird 1 bis 2 cm lateral und 2 bis 3 cm caudal vom ersten ausgeführt. Der dritte Einschnitt wird 3 cm lateral und dorsal vom Anus ausgeführt. Dieselben Einschnitte werden auf der gegenüberliegenden Seite ausgeführt.

Das Führungselement ist nach einer Variante der Erfindung gekrümmt ausgebildet, wodurch es einfacher durch den äußeren Einschnitt und das umliegende Gewebe in den Hohlraum des menschlichen Körpers, insbesondere der Vagina, der Patientin führen lässt. Das Führungselement besteht beispielsweise aus einem Metall, insbesondere aus Edelstahl.

Zur besseren Handhabung umfasst das Führungselement nach einer erfindungsgemäßen Variante einen Handgriff.

Nachfolgend wird die Erfindung anhand der in den Figuren dargestellten Ausführungsbeispiele näher beschrieben. Es zeigen:
- Fig. 1: eine schematische Ansicht eines Führungselements einer erfindungsgemäßen Anordnung zum Einsetzen eines chirurgischen Implantats mit mindestens einem Implantatansatzabschnitt,
- Fig. 2: eine schematische Ansicht einer Kanüle einer erfindungsgemäßen Anordnung zum Einsetzen eines chirurgischen Implantats mit mindestens einem Implantatansatzabschnitt,
- Fig. 3: eine Schnittansicht durch die Kanüle aus Fig. 2,
- Fig. 4: eine schematische Ansicht einer Zurückholeinrichtung einer erfindungsgemäßen Anordnung zum Einsetzen eines chirurgischen Implantats mit mindestens einem Implantatansatzabschnitt,
- Fig. 5: eine Querschnittsansicht durch die Zurückholeinrichtung aus Fig. 4,
- Fig. 6: Detailansichten von Fangelementen einer Zurückholeinrichtung,
- Fig. 7: eine alternative Ausgestaltung einer Zurückholeinrichtung einer erfindungsgemäßen Anordnung zum Einsetzen eines chirurgischen Implantats mit mindestens einem Implantatansatzabschnitt,
- Fig. 8 bis 18: Details einer Anwendung einer erfindungsgemäßen Anordnung.

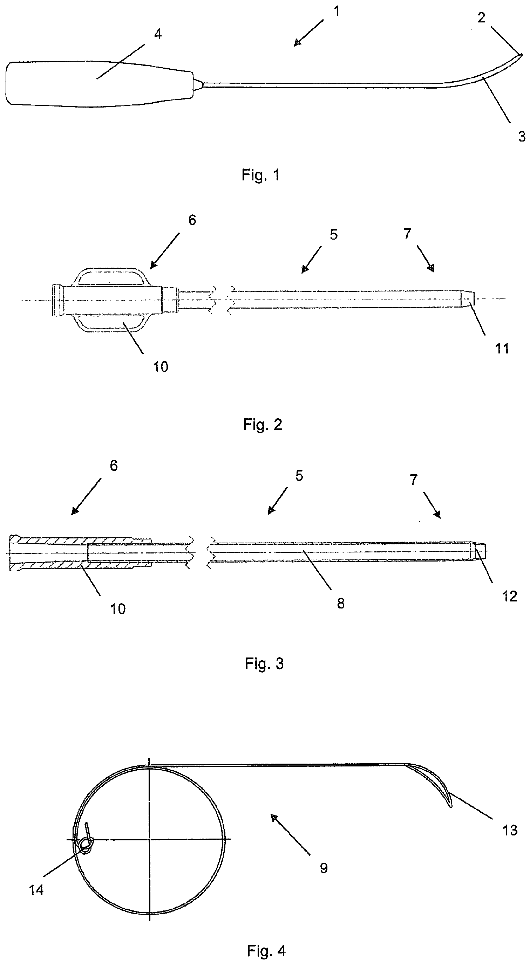

Fig. 1 zeigt eine schematische Ansicht eines Führungselements 1 einer erfindungsgemäßen Anordnung zum Einsetzen eines chirurgischen Implantats mit mindestens einem Implantatansatzabschnitt 17. Das Führungselement 1 weist am distalen Ende ein Gewebedurchbohrende 2 auf und ist so dimensioniert, dass es während der Anwendung durch die Beckenhöhle einer Patientin von einem äußeren Einschnitt 16 durch das umliegende Gewebe in einen Hohlraum des menschlichen Körpers, insbesondere der Vagina einer Patientin, verläuft. Das Führungselement 1 weist weiterhin eine Krümmung 3 auf, insbesondere im distalen Bereich des Führungselements 1. Zur besseren Handhabung umfasst das Führungselement 1 am proximalen Ende einen Handgriff 4. Vorzugsweise besteht das Führungselement 1 aus einem Metall, insbesondere aus Edelstahl.

In Fig. 2 ist eine schematische Ansicht einer Kanüle 5 einer erfindungsgemäßen Anordnung zum Einsetzen eines chirurgischen Implantats mit mindestens einem Implantatansatzabschnitt 17 dargestellt. Die Kanüle 5 hat ein proximales Ende 6, ein distales Ende 7 und einen zwischen dem proximalen Ende 6 und dem distalen Ende 7 verlaufenden Kanal 8. Der Kanal 8 der Kanüle 5 ist dabei derart ausgebildet, dass das Führungselement 1 darin gleitfähig aufnehmbar ist. Die Kanüle 5 mit dem darin gleitfähig aufgenommenen Führungselement 1 ist so dimensioniert, dass die Kanüle 5 wie das Führungselement 1 während der Anwendung durch die Beckenhöhle einer Patientin von einem äußeren Einschnitt 16 durch das umliegende Gewebe in einen Hohlraum des menschlichen Körpers, insbesondere der Vagina einer Patientin, verläuft. Der Kanal 8 durch die Kanüle 5 ist weiterhin für einen Durchgang einer beispielsweise in Fig. 4 dargestellten Zurückholeinrichtung 9 zusammen mit einem eingefangenen Implantatansatzabschnitt 17 ausgebildet. Die Kanüle 5 ist weiterhin derart ausgebildet, dass sie sich in einem ersten Zustand, in welchem das Führungselement 1 innerhalb des Kanals 8 der Kanüle 5 angeordnet ist, an die Form des Führungselements 1 anpasst. In einem zweiten Zustand, in welchem die Zurückholeinrichtung 9 innerhalb des Kanals 8 der Kanüle 5 angeordnet ist, ist die Kanüle 5 im Wesentlichen gerade ausgebildet.

Dass die Kanüle 5 in dem zweiten Zustand im Wesentlichen gerade ausgebildet ist, bedeutet im Sinne der Erfindung, dass zumindest das distale Ende 7 der Kanüle 5, welches in den Hohlraum des menschlichen Körpers, insbesondere der Vagina einer Patientin, austritt, gerade ausgebildet ist.

Wie insbesondere der in Fig. 3 dargestellten Schnittansicht der Kanüle 5 aus Fig. 2 entnommen werden kann, ist der Durchmesser des Kanals 8 der Kanüle 5 am proximalen Ende 6 erweitert. Dies erleichtert das Einführen des Führungselements 1 oder der Zurückholeinrichtung 9 in den Kanal 8 der Kanüle 5.

Am proximalen Ende 6 der Kanüle 5 ist ein Handgriff 10 angeordnet, wodurch sich die Kanüle 5 während der Anwendung einfacher handhaben lässt. Weiterhin verhindert der Handgriff 10, dass die Kanüle 5 während der Anwendung vollständig in den äußeren Einschnitt 16 eintritt. Zweckmäßigerweise besteht der Handgriff 10 der Kanüle 5 aus einem starren Material, beispielsweise einem starren Kunststoff oder einem Metall.

In dem in den Figuren 2 und 3 dargestellten Ausführungsbeispiel verringert sich der Außendurchmesser der Kanüle 5 am distalen Ende 7 konisch 11. Dadurch wird ein fließender Übergang zwischen der in dem Kanal 8 der Kanüle 5 eingeführten Führungselement 1 und der Außenwandung der Kanüle 5 erzielt, was insbesondere beim Durchführen der Kanüle 5 zusammen mit dem Führungselement 1 von dem äußeren Einschnitt 16 durch das umliegende Gewebe in den Hohlraum des menschlichen Körpers, insbesondere der Vagina, erleichtert.

Weiterhin verringert sich der Innendurchmesser 12 der Kanüle 5 am distalen Ende 7 auf einen Durchmesser der etwas geringer ist als der Außendurchmesser des Führungselements 1 am distalen Ende, jedoch größer als der Durchmesser des Gewebedurchbohrendes 2 des Führungselements 1. Dadurch wird die Kanüle 5 mittels einer reibschlüssigen Verbindung beim Einführen in den äußeren Einschnitt 16 relativ zu dem Führungselement 1 fixiert.

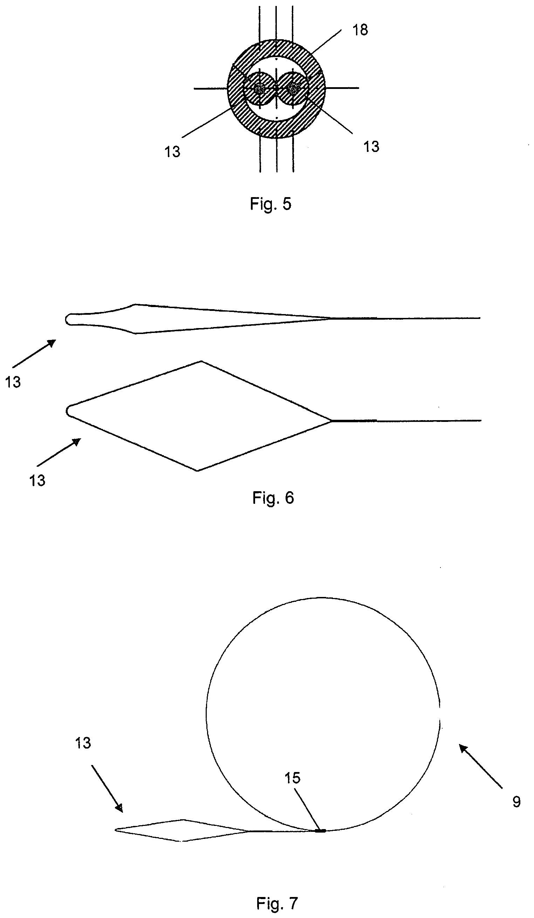

Zweckmäßigerweise ist die Kanüle 5 aus wenigstens einem Kunststoff hergestellt, vorzugsweise aus einem flexiblen Kunststoff, so dass sich die Kanüle 5 in einem ersten Zustand, in welchem das Führungselement 1 innerhalb des Kanals 8 der Kanüle 5 angeordnet ist, an die Form des Führungselements 1 anpasst und dass die Kanüle 5 in einem zweiten Zustand, in welchem die Zurückholeinrichtung 9 innerhalb des Kanals 8 der Kanüle 5 angeordnet ist, im Wesentlichen gerade ausgebildet ist. Die Kanüle 5 kann auch aus unterschiedlichen Kunststoffen hergestellt sein, um in verschiedenen Bereichen der Kanüle 5 unterschiedliche Steifigkeiten zu erzielen. Zur Verbesserung der Steifigkeit bzw. als Knickschutz sind innerhalb des Kunststoffs ein oder mehrere Versteifungselemente, beispielsweise Metalldrähte, eingearbeitet werden.
In Fig. 4 ist eine schematische Ansicht einer Ausführungsform einer Zurückholeinrichtung 9 einer erfindungsgemäßen Anordnung zum Einsetzen eines chirurgischen Implantats mit mindestens einem Implantatansatzabschnitt 17 dargestellt. Am distalen Ende der Zurückholeinrichtung 9 ist ein Fangelement 13 angeordnet, zum Einfangen des mindestens einen Implantatansatzabschnitts 17 des chirurgischen Implantats.
In der in Fig. 4 dargestellten Ausführungsform ist das Fangelement 13 der Zurückholeinrichtung 9 als Schlinge ausgebildet.
Das Fangelement 13 der Zurückholeinrichtung 9 besteht beispielsweise aus einem Metall, einem Kunststoff oder einem Formgedächtnismaterial, insbesondere aus Edelstahl oder Nitinol.
Zweckmäßigerweise ist das Fangelement 13 der Zurückholeinrichtung 9 derart ausgebildet, dass es sich nach dem Austreten aus dem distalen Ende 7 des Kanals 8 der Kanüle 5 relativ zu der durch den Kanal 8 der Kanüle 5 verlaufenden Längsachse abwinkelt, vorzugsweise um etwa 90°.
Gemäß der in Fig. 4 dargestellten Ausführungsform weist die Zurückholeinrichtung 9 im Bereich des proximalen Endes einen Knoten 14 auf. Fig. 5 zeigt eine Querschnittsansicht der Zurückholeinrichtung 9 aus Fig. 4. Dieser ist zu entnehmen, dass die Zurückholeinrichtung 9 aus einem länglichen hohlen Element 18 besteht. Innerhalb des hohlen Elements 18 ist das Fangelement 13 angeordnet, welches aus einem Draht besteht, welcher am distalen Ende der Zurückholeinrichtung 9 eine Schlinge ausbildet. Der Knoten 14 am proximalen Ende der Zurückholeinrichtung 9 verhindert dabei, dass sich das Fangelement 13 ungewollt relativ zu der Zurückholeinrichtung 9 bewegt.

Ist das Fangelement 13 als Schlinge ausgebildet, kann die Schlinge mittels einer distalen Manschette ausgebildet werden, in welcher ein Ende des die Schlinge ausbildenden Drahts fixiert ist, während ein anderer Bereich des die Schlinge ausbildenden Drahts gleitfähig in der distalen Manschette gelagert ist.

In Fig. 6 sind unterschiedliche als Schlingen ausgebildete Fangelemente 13 dargestellt. Die Fangelemente 13 bestehen aus einem Draht, bei welchem ein Ende des Drahts im Bereich des distalen Endes fest mit einem anderen Bereich des Drahts verbunden ist, so dass ab diesem Bereich die distale Schlinge des Fangelements 13 ausgebildet wird. Die in Fig. 6 dargestellten Fangelemente 13 können mit einem hohlen Element 18 zusammen als Zurückholeinrichtung 9 eingesetzt werden, aber auch ohne ein zusätzliches hohles Element 18 als Zurückholeinrichtung 9 verwendet werden.

Fig. 7 zeigt eine alternative Ausgestaltung einer Zurückholeinrichtung 9 einer erfindungsgemäßen Anordnung zum Einsetzen eines chirurgischen Implantats mit mindestens einem Implantatansatzabschnitt 17. Die Zurückholeinrichtung 9 wird in dem dargestellten Ausführungsbeispiel durch einen Metalldraht ausgebildet. Ein Ende des Drahts ist im Bereich des distalen Endes fest mit einem anderen Bereich des Drahts verbunden ist, so dass ab diesem Bereich eine distale Schlinge als Fangelement 13 ausgebildet wird. Das proximale Ende des Drahts ist an einer Manschette 15 fixiert, während ein anderer Bereich des Drahts gleitfähig in der Manschette 15 gelagert ist. Dadurch lässt sich die Gesamtlänge der Zurückholeinrichtung 9 anpassen. Beim Einführen der Zurückholeinrichtung 9 kann die am proximalen Ende ausgebildete Schlaufe verringert und beim Herausziehen vergrößert werden. Dadurch verringert sich der proximale Platzbedarf der Zurückholeinrichtung 9, insbesondere wird die Gefahr eines Verhakens mit anderen während der Operation verwendeten Gegenständen und/oder bei der Operation anwesenden Personen verringert.

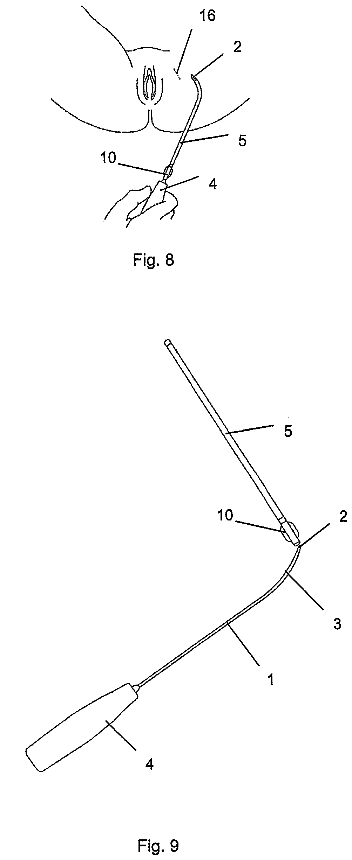

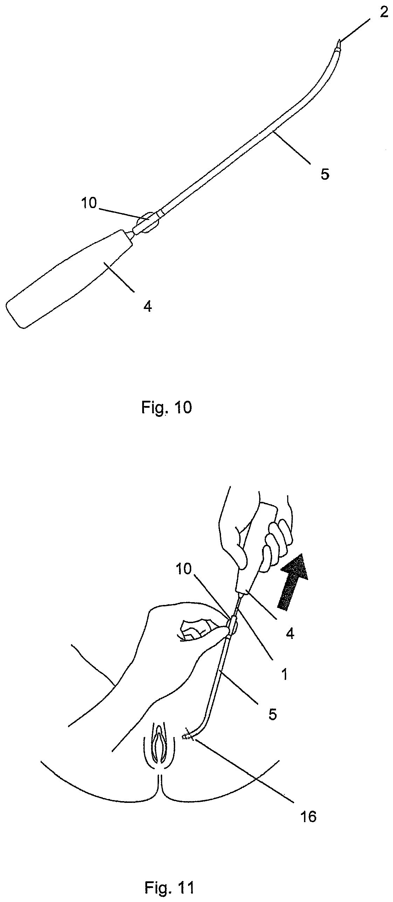

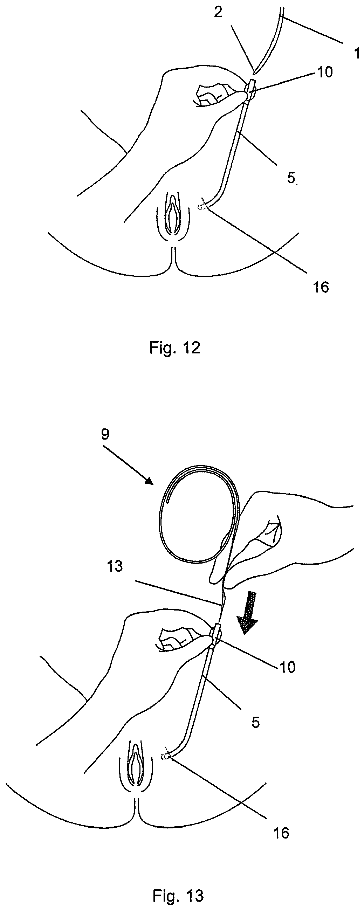

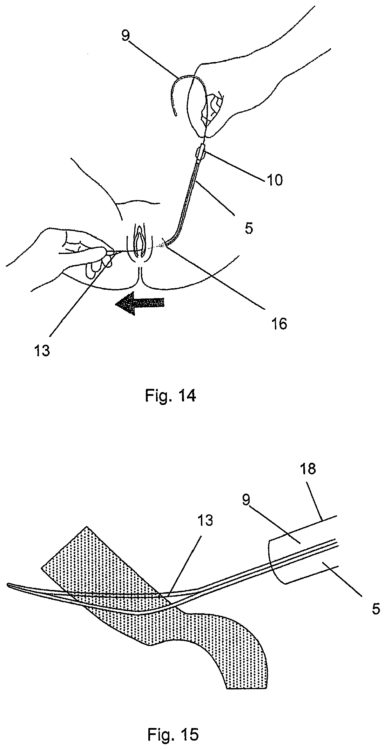

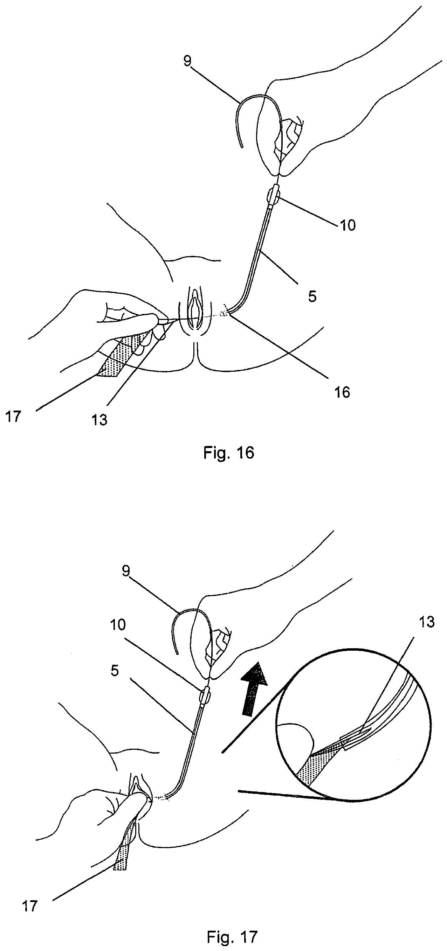

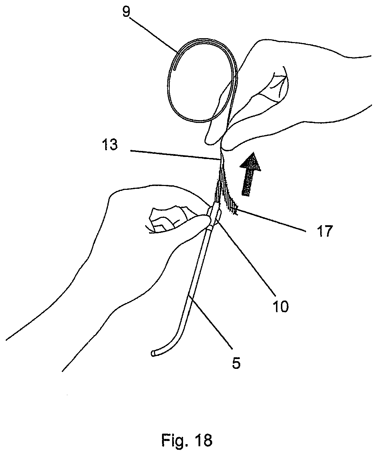

Zweckmäßigerweise ist das Implantat ein Netz zur Beckenbodenreparatur, ein Netz zur Zystozelenreparatur oder ein Netz zur Rektozelenreparatur. Derartige Implantate sind im Detail in der EP 1 804 715 B1 beschreiben. Ein Implantat zur Behandlung einer Harninkontinenz ist beispielsweise in der EP 1 333 774 B1 offenbart.
Bei dem Einsetzen von Netzimplantaten mit mindestens einem bandartigen Implantatansatzabschnitt 17 zur Beckenbodenrekonstruktion wird zunächst ein äußerer Einschnitt 16 im Bereich des Implantationsorts ausgeführt. Der äußere Einschnitt 16 ist insbesondere der Fig. 8 zu entnehmen.
Zum Fixieren des Netzimplantats wird in einem ersten Schritt das Führungselement 1 durch den Kanal 8 der Kanüle 5 geführt, bis das Gewebedurchbohrende 2 aus dem distalen Ende 7 der Kanüle 5 austritt, wie in Fig. 9 und 10 dargestellt. Nachfolgend wird das Führungselement 1 zusammen mit der Kanüle 5 durch den äußeren Einschnitt 16 eingeführt bis das Gewebedurchbohrende 2 des Führungselements 1 und das distale Ende 7 der Kanüle 5 durch das umliegende Gewebe in den Hohlraum des menschlichen Körpers, insbesondere der Vagina der Patientin, austritt (siehe Fig. 8). Als nächstes wird das Führungselement 1 aus der Kanüle 5 entfernt, wobei die Lage der Kanüle 5 nicht verändert wird. Dieser Schritt ist in den Figuren 11 und 12 dargestellt.
Durch den Kanal 8 der Kanüle 5 wird nun die Zurückholeinrichtung 9 in die Patientin eingeführt, wie in den Figuren 13 und 14 dargestellt. Der Implantatansatzabschnitt 17 wird mittels des Fangelements 13 der Zurückholeinrichtung 9 eingefangen. Sobald der Implantatansatzabschnitt 17 mit dem Fangelement 13 der Zurückholeinrichtung 9 gekoppelt ist, wird die Zurückholeinrichtung 9 aus dem Kanal 8 der Kanüle 5 entfernt, so dass sich der gekoppelte Implantatansatzabschnitt 17 durch den Kanal 8 der Kanüle 5 erstreckt, wie in den Figuren 15 bis 18 dargestellt. Aus Gründen der Übersichtlichkeit wurde in der Figur 18 der Körper der Patientin nicht dargestellt.
Anschließend wird die Kanüle 5 entfernt, wobei der Implantatansatzabschnitt 17 aus dem Kanal 8 der Kanüle 5 entfernt wird, so dass sich der Implantatansatzabschnitt 17 weiterhin zwischen der Vagina der Patientin und dem äußeren Einschnitt erstreckt. Weist das Netzimplantat mehrere Implantatansatzabschnitte 17 auf, wird für jeden Implantatansatzabschnitt 17 jeweils ein äußeren Einschnitt ausgeführt und jeweils ein Implantatansatzabschnitt 17 nach dem zuvor beschriebenen Verfahren angeordnet. Nach dem Einsetzen des Implantats verwachsen die Implantatansatzabschnitte 17 mit dem umliegenden Gewebe, wodurch das Implantat an der gewünschten Stelle fixiert wird.

### Bezugszeichenliste

- 1: Führungselement
- 2: Gewebedurchbohrende
- 3: Krümmung Führungselement
- 4: Handgriff Führungselement
- 5: Kanüle
- 6: Proximales Ende Kanüle
- 7: Distales Ende Kanüle
- 8: Kanal durch Kanüle
- 9: Zurückholeinrichtung
- 10: Handgriff Kanüle
- 11: Verringerung äußerer Durchmesser der Kanüle
- 12: Verringerung Innendurchmesser der Kanüle
- 13: Fangelement
- 14: Knoten am proximalen Ende der Zurückholeinrichtung
- 15: Proximale Manschette
- 16: Äußerer Einschnitt
- 17: Implantatansatzabschnitt
- 18: Hohles Element

## Patentansprüche

1. Anordnung zum Einsetzen eines chirurgischen Implantats mit mindestens einem Implantatansatzabschnitt (17), umfassend:
ein Führungselement (1) mit einem distalen Gewebedurchbohrende (2);
eine Kanüle (5) mit einem proximalen Ende (6), einem distalen Ende (7) und einem zwischen dem proximalen (6) und dem distalen Ende (7) verlaufenden Kanal (8), wobei das Führungselement (1) in dem Kanal (8) gleitfähig aufnehmbar ist; und
eine Zurückholeinrichtung (9) mit einem Fangelement (13) an einem distalen Ende der Zurückholeinrichtung (13), zum Einfangen des mindestens einen Implantatansatzabschnitts (17) des chirurgischen Implantats;
wobei die Kanüle (5) mit dem darin gleitfähig aufgenommenen Führungselement (1) so dimensioniert ist, dass sie durch die Beckenhöhle einer Patientin von einem äußeren Einschnitt (16) durch das umliegende Gewebe in einen Hohlraum des menschlichen Körpers, insbesondere der Vagina einer Patientin, verläuft, und der Kanal (8) der Kanüle (5) für einen Durchgang der Zurückholeinrichtung (9) und des eingefangenen Implantatansatzabschnitts (17) ausgebildet ist,
wobei sich die Kanüle (5) in einem ersten Zustand, in welchem das Führungselement (1) innerhalb des Kanals (8) der Kanüle (5) angeordnet ist, an die Form des Führungselements (1) anpasst und dass das distale Ende der Kanüle (5), welches in den Hohlraum des menschlichen Körpers, insbesondere der Vagina, austritt, in einem zweiten Zustand bei dem Einsetzen des chirurgischen Implantats, in welchem die Zurückholeinrichtung (9) innerhalb des Kanals (8) der Kanüle (5) angeordnet ist, gerade ausgebildet ist,
**dadurch gekennzeichnet, dass**
die Kanüle (5) aus wenigstens einem Kunststoff mit einem innenliegenden Versteifungselement besteht.

2. Anordnung nach Anspruch 1, **dadurch gekennzeichnet, dass** der Durchmesser des Kanals (8) der Kanüle (5) am proximalen Ende (6) der Kanüle (5) erweitert ist.

3. Anordnung nach Anspruch 1 oder Anspruch 2, **dadurch gekennzeichnet, dass** die Kanüle (5) am proximalen Ende (6) wenigstens einen Handgriff (10) umfasst.

4. Anordnung nach einem der Ansprüche 1 bis 3, **dadurch gekennzeichnet, dass** sich der Außendurchmesser der Kanüle (5) am distalen Ende (7) konisch verringert (11).

5. Anordnung nach einem der Ansprüche 1 bis 4, **dadurch gekennzeichnet, dass** das Fangelement (13) der Zurückholeinrichtung (9) als Schlinge oder Haken ausgebildet ist.

6. Anordnung nach einem der Ansprüche 1 bis 5, **dadurch gekennzeichnet, dass** das Fangelement (13) der Zurückholeinrichtung (9) aus einem Metall, einem Kunststoff oder einem Formgedächtnismaterial besteht, insbesondere aus Edelstahl oder Nitinol.

7. Anordnung nach einem der Ansprüche 1 bis 6, **dadurch gekennzeichnet, dass** das Fangelement (13) derart ausgebildet ist, dass es sich beim Austreten aus dem distalen Ende (7) des Kanals (8) der Kanüle (5) relativ zu der durch den Kanal (8) der Kanüle (5) verlaufenden Längsachse abwinkelt, vorzugsweise um etwa 90°.

8. Anordnung nach einem der Ansprüche 1 bis 7, **dadurch gekennzeichnet, dass** die Zurückholeinrichtung (9) im Bereich des proximalen Endes einen Knoten (14) aufweist.

9. Anordnung nach einem der Ansprüche 1 bis 8, **dadurch gekennzeichnet, dass** das Fangelement (13) als Schlinge ausgebildet ist, wobei die Schlinge mittels einer distalen Manschette ausgebildet wird, in welcher ein Ende des die Schlinge bildenden Drahts fixiert ist, während ein anderer Bereich des Drahts gleitfähig in der distalen Manschette gelagert ist.

10. Anordnung nach einem der Ansprüche 1 bis 9, **dadurch gekennzeichnet, dass** das proximale Ende des Fangelements (13) an einer proximalen Manschette (15) fixiert ist, während ein anderer Bereich des Fangelements (13) gleitfähig in der proximalen Manschette (15) gelagert ist.

11. Anordnung nach einem der Ansprüche 1 bis 10, **dadurch gekennzeichnet, dass** der äußere Einschnitt ein Einschnitt in dem Unterbauch, im Anusbereich oder in dem Oberschenkel in der Nähe des foramen obturatum ist.

12. Anordnung nach einem der Ansprüche 1 bis 11, **dadurch gekennzeichnet, dass** das Führungselement (1) gekrümmt (3) ausgebildet ist.

13. Anordnung nach einem der Ansprüche 1 bis 12, **dadurch gekennzeichnet, dass** das Führungselement (1) aus einem Metall besteht, vorzugsweise aus Edelstahl.

14. Anordnung nach einem der Ansprüche 1 bis 13, **dadurch gekennzeichnet, dass** das Führungselement (1) einen Handgriff (4) umfasst.

## Claims

1. Arrangement for inserting a surgical implant with at least one implant attachment portion (17), comprising:
a guide element (1) having a distal tissue piercing end (2);
a cannula (5) having proximal end (6), a distal end (7) and a channel (8) extending between the proximal end (6) and distal end (7), wherein the guide element (1) is slidably receivable in the channel (8); and
a retrieval device (9) having a capture element (13) at a distal end of the retrieval device (13) for capturing the at least one implant attachment portion (17) of the surgical implant;
wherein the cannula (5), with the guide element (1) slidably received therein, being dimensioned to pass through the pelvic cavity of a patient from an external incision (16) through the surrounding tissue into a cavity of the human body, particularly a patient's vagina, and the channel (8) of the cannula (5) is designed for a passage of the retrieval device (9) and the captured implant attachment portion (17),
wherein the cannula (5) in a first state, in which the guide element (1) is disposed within the channel (8) of the cannula (5), conforms to the shape of the guide element (1), and that the distal end of the cannula (5), which extends into the cavity of the human body, particularly the vagina, is substantially straight in a second state during insertion of the surgical implant, in which the retrieval device (9) is arranged in the channel (8) of the cannula (5),
**characterized in that**
the cannula (5) consists of at least one plastic with an internal stiffening element.

2. Arrangement according to claim 1, **characterized in that** the diameter of the channel (8) of the cannula (5) at the proximal end (6) of the cannula (5) is enlarged.

3. Arrangement according to claim 1 or claim 2, **characterized in that** the cannula (5) at the proximal end (6) comprises at least one handle (10).

4. Arrangement according to one of claims 1 to 3, **characterized in that** the outer diameter of the cannula (5) at the distal end (7) is conically reduced.

5. Arrangement according to one of claims 1 to 4, **characterized in that** the capture element (13) of the retrieval device (9) is designed as a loop or hook.

6. Arrangement according to one of claims 1 to 5, **characterized in that** the capture element (13) of the retrieval device (9) consist of a metal, a plastic or shape memory material, particularly of stainless steel or Nitinol.

7. Arrangement according to one of claims 1 to 6, **characterized in that** the capture element (13) is designed such that when it emerges from the distal end (7) of the channel (8) of the cannula (5) pivots relative to the longitudinal axis extending through the channel (8) of the cannula (5), particularly by about 90°.

8. Arrangement according to one of claims 1 to 7, **characterized in that** the retrieval device (9) in the region of the proximal end has a knot (14).

9. Arrangement according to one of claims 1 to 8, **characterized in that** the capture element (13) is formed as a loop, wherein the loop is formed by means of a distal cuff, in which one end of the wire building the loop is fixed, whereas another portion of the wire is slidably arranged in the distal cuff.

10. Arrangement according to one of claims 1 to 9, **characterized in that** the proximal end of the capture element (13) is fixed at a proximal cuff (15), whereas another portion of the capture element (13) is slidably arranged in the proximal cuff (15).

11. Arrangement according to one of claims 1 to 10, **characterized in that** the external incision is an incision in the lower abdomen, in the anus area or in the thigh near the foramen obturatum.

12. Arrangement according to one of claims 1 to 11, **characterized in that** the guide element (1) is curved (3).

13. Arrangement according to one of claims 1 to 12, **characterized in that** the guide element (1) consist of metal, particularly of stainless steel.

14. Arrangement according to one of claims 1 to 13, **characterized in that** the guide element (1) comprises a handle (4).

## Revendications

1. Agencement permettant d'insérer un implant chirurgical avec au moins une partie de fixation d'implant (17), comprenant :
un élément de guidage (1) comportant une extrémité distale de perforation de tissu (2) ;
une canule (5) comportant une extrémité proximale (6), une extrémité distale (7) et un canal (8) s'étendant entre l'extrémité proximale (6) et l'extrémité distale (7), l'élément de guidage (1) pouvant être reçu de manière coulissante dans le canal (8) ; et
un dispositif d'extraction (9) comportant un élément de capture (13) au niveau d'une extrémité distale du dispositif d'extraction (13) permettant de capturer l'au moins une partie de fixation d'implant (17) de l'implant chirurgical ;
dans lequel la canule (5), grâce à l'élément de guidage (1) reçu de manière coulissante dans celle-ci, est dimensionnée pour traverser la cavité pelvienne d'un patient à partir d'une incision externe (16) à travers le tissu environnant dans une cavité du corps humain, en particulier le vagin d'une patiente, et le canal (8) de la canule (5) est conçu pour un passage du dispositif d'extraction (9) et de la partie de fixation d'implant (17) capturée,
dans lequel la canule (5), dans un premier état, dans lequel l'élément de guidage (1) est disposé à l'intérieur du canal (8) de la canule (5), épouse la forme de l'élément de guidage (1), et l'extrémité distale de la canule (5), qui s'étend dans la cavité du corps humain, en particulier le vagin, est sensiblement droite dans un second état pendant l'insertion de l'implant chirurgical, dans lequel le dispositif d'extraction (9) est agencé dans le canal (8) de la canule (5),
**caractérisé en ce que**
la canule (5) est constituée d'au moins un plastique doté d'un élément de renforcement interne.

2. Agencement selon la revendication 1, **caractérisé en ce que** le diamètre du canal (8) de la canule (5) au niveau de l'extrémité proximale (6) de la canule (5) est élargi.

3. Agencement selon la revendication 1 ou la revendication 2, **caractérisé en ce que** la canule (5) au niveau de l'extrémité proximale (6) comprend au moins une poignée (10).

4. Agencement selon l'une des revendications 1 à 3, **caractérisé en ce que** le diamètre externe de la canule (5) au niveau de l'extrémité distale (7) est réduit de manière conique.

5. Agencement selon l'une des revendications 1 à 4, **caractérisé en ce que** l'élément de capture (13) du dispositif d'extraction (9) se présente sous la forme d'une boucle ou d'un crochet.

6. Agencement selon l'une des revendications 1 à 5, **caractérisé en ce que** l'élément de capture (13) du dispositif d'extraction (9) est constitué d'un matériau métallique, plastique ou à mémoire de forme, notamment en acier inoxydable ou en Nitinol.

7. Agencement selon l'une des revendications 1 à 6, **caractérisé en ce que** l'élément de capture (13) se présente de telle sorte que lorsqu'il émerge de l'extrémité distale (7) du canal (8) de la canule (5), il pivote par rapport à l'axe longitudinal s'étendant à travers le canal (8) de la canule (5), notamment d'environ 90°.

8. Agencement selon l'une des revendications 1 à 7, **caractérisé en ce que** le dispositif d'extraction (9) dans la région de l'extrémité proximale comporte un noeud (14).

9. Agencement selon l'une des revendications 1 à 8, **caractérisé en ce que** l'élément de capture (13) est sous forme de boucle, la boucle étant formée au moyen d'un manchon distal, dans lequel une extrémité du fil de construction de la boucle est fixe, tandis qu'une autre partie du fil est agencée de manière coulissante dans le manchon distal.

10. Agencement selon l'une des revendications 1 à 9, **caractérisé en ce que** l'extrémité proximale de l'élément de capture (13) est fixée au niveau d'un manchon proximal (15), tandis qu'une autre partie de l'élément de capture (13) est agencée de manière coulissante dans le manchon proximal (15).

11. Agencement selon l'une des revendications 1 à 10, **caractérisé en ce que** l'incision externe est une incision de l'abdomen inférieur, dans la zone de l'anus ou dans la cuisse à proximité du foramen obturé.

12. Agencement selon l'une des revendications 1 à 11, **caractérisé en ce que** l'élément de guidage (1) est incurvé (3).

13. Agencement selon l'une des revendications 1 à 12, **caractérisé en ce que** l'élément de guidage (1) est constitué de métal, notamment d'acier inoxydable.

14. Agencement selon l'une des revendications 1 à 13, **caractérisé en ce que** l'élément de guidage (1) comprend une poignée (4).
